# EUROPEAN PATENT SPECIFICATION

(11) **EP 3 215 078 B1**
(45) Date of publication and mention of the grant of the patent: **06.04.2022**
(21) Application number: 15857869.0
(22) Date of filing: 05.11.2015
(51) Int. Cl.: A61F 7/00, F04B 43/12, F04B 43/08, F04B 43/00, A61F 7/12

(54) **EASY LOADING HIGH PERFORMANCE PERISTALTIC PUMP**
LEICHT ZU LADENDE, HOCHLEISTUNGSFÄHIGE PERISTALTIKPUMPE
POMPE PÉRISTALTIQUE HAUTE PERFORMANCE À CHARGEMENT AISÉ

(30) Priority: 06.11.2014 US 201414534718; 01.04.2015 US 201514676682
(43) Date of publication of application: 13.09.2017
(60) Divisional application: 22159271.0
(73) Proprietor: ZOLL Circulation, Inc., San Jose, CA 95131 (US)
(72) Inventor: HENDRICKS, Austin Reid, Union City, California 94587 (US); PAMICHEV, Christo Petrov, Cupertino, California 95014 (US); GURUKULA, Venkata Vishnu, Mountain View, California 94040 (US); DABROWIAK, Jeremy Thomas, Santa Clara, California 95054 (US)
(74) Representative: Potter Clarkson
(86) International application number: PCT/US2015/059244
(87) International publication number: WO 2016/073721

(56) References cited:
- EP-A1- 0 663 529
- EP-B1- 0 663 529
- EP-B1- 0 663 529
- DE-A1-102009 050 053
- US-A- 5 759 017
- US-A1- 2005 053 502
- US-A1- 2005 069 437
- US-A1- 2010 129 248
- US-A1- 2012 148 415
- US-A1- 2014 081 202
- US-B2- 8 272 857

## Description

### TECHNICAL FIELD

The present application relates generally to easy loading high performance peristaltic pumps.

### BACKGROUND

Patient temperature control systems have been introduced to prevent fever in patients in the neuro ICU due to suffering from sub-arachnoid hemorrhage or other neurologic malady such as stroke. Also, such systems have been used to induce mild or moderate hypothermia to improve the outcomes of patients suffering from such maladies as stroke, cardiac arrest, myocardial infarction, traumatic brain injury, and high intracranial pressure. Examples of intravascular heat exchange catheters are disclosed in U.S. Patent Nos. 7,914,564, 6,416,533, 6,409,747, 6,405,080, 6,393,320, 6,368,304, 6,338,727, 6,299,599, 6,290,717, 6,287,326, 6,165,207, 6,149,670, 6,146,411, 6,126,684, 6,306,161, 6,264,679, 6,231,594, 6,149,676, 6,149,673, 6,110,168, 5,989,238, 5,879,329, 5,837,003, 6,383,210, 6,379,378, 6,364,899, 6,325,818, 6,312,452, 6,261,312, 6,254,626, 6,251,130, 6,251,129, 6,245,095, 6,238,428, 6,235,048, 6,231,595, 6,224,624, 6,149,677, 6,096,068, 6,042,559, 8,888,729, and USPPs 2013/0178923, 2013/0079855, 2013/0079856, 2014/0094880, 2014/0094882, 2014/0094883.

External patient temperature control systems may be used. Such systems are disclosed in U.S. Patent Nos. 6,827,728, 6,818,012, 6,802,855, 6,799,063, 6,764,391, 6,692,518, 6,669,715, 6,660,027, 6,648,905, 6,645,232, 6,620,187, 6,461,379, 6,375,674, 6,197,045, and 6,188,930 (collectively, "the external pad patents").

In general, in all of the intravascular and external patient temperature control solutions, the temperature of the working fluid flowing through the catheter or pad is regulated by a heat exchange console based on feedback provided by the patient's actual body temperature, typically core body temperature as may be variously measured rectally, esophageally, tympanic ear temperature, blood temperature in, e.g., the vena cava, etc. The working fluid temperature is regulated by thermally coupling the working fluid to heating and/or cooling elements in the console. In many cases, the working fluid is forced in a closed fluid circuit path (including the console and the catheter or pad) by a peristaltic pump acting on tubing, e.g., pump tubing or IV tubing, in the fluid circuit path. DE 102009050053 describes a peristaltic pump comprising a head with pinch rollers which can be moved between a working position where at least one pinch roller clamps a pump hose against a hose bed and a release position providing free space between the pinch rollers and the hose bed. An eccentric drive and knurled wheel may be used to move the head between the working position and release position. US 2012/0148415 describes a pump rotor for a peristaltic hose pump and an actuating device by which the pump rotor can be actuated hydraulically and/or pneumatically. US 2005/0069437 describes a peristaltic pump having a pump head with rollers, and a backing plate which cooperates with the pump head to pinch a length of tubing between the rollers and the backing plate surface. US 2005/0053502 also describes a peristaltic pump.

### SUMMARY

As understood herein, peristaltic pumps typically include a rotor for revolving one or more rollers against an IV tube to force fluid through the tube by peristalsis, and an arcuate raceway against which the tube is urged by the rollers. The ease by which the tube can be loaded between the rollers and raceway competes with the performance of the pump: an easier to load pump typically has lower performance, whereas a higher performance pump (with higher pumping pressure and fluid flow) usually entails more complex loading of the tube. This is because in easy to load pumps, the raceway is typically movable away from the rollers to facilitate easily placing the tube between the rollers and raceway, but higher performance pumps require longer raceways (greater than 180 degrees of arc) that are generally not movable away from the pump, complicating the task of loading the tube (which for high performance applications is relatively thick and inflexible compared to low performance tubes) between the rollers and raceway.

The invention provides a pump assembly according to claim 1. Further embodiments of the invention are illustrated in the dependent claims. More generally described in this disclosure is a pump which has an arcuate raceway with a concave inner surface extending through an arc of at least one hundred eighty degrees (180°). The arc defines a midpoint, and a rotor faces the inner surface of the raceway and is both rotatable relative to the raceway and translationally mounted relative to the raceway between a pump position, wherein the rotor is spaced from the midpoint a first distance, and a tube load position, wherein the rotor is spaced from the midpoint a second distance greater than the first distance. A motor is coupled to the rotor to rotate the rotor. Plural rollers are arranged on the rotor to contact tubing disposed between the rotor and the raceway at least when the rotor is in the pump position. The motor is prevented from stopping at least one angular position in which at least one roller is in a predetermined location.

If desired, the concave inner surface of the raceway may extend through an arc of between one hundred eighty degrees (180°) and two hundred degrees (200°). The rollers can include at least one drive roller (46) on the rotor. The drive roller (46) can have a cylindrical outer surface, with the entire outer surface being configured to urge against a tube disposed between the rotor and the raceway when the rotor is in the pump position and is rotated. The drive roller (46) has no flanges with peripheries extending beyond the cylindrical outer surface. At least one guide roller may be on the rotor and may have a cylindrical outer surface and top and bottom flanges defining respective peripheries extending beyond the cylindrical outer surface of the guide roller. With this structure, a tube disposed between the rotor and the raceway is received on the cylindrical outer surface of the guide roller between the flanges when the rotor is in the pump position and is rotated. Plural drive rollers and plural guide rollers may be provided; in some implementations, two and only two drive rollers and two and only two guide rollers are provided.

In examples, a motor mount supports the motor and rotor and a positioning mechanism is coupled to the motor mount and is manipulable by a person to move the motor mount to thereby move the rotor between the pump position and the tube load position. The motor mount can be parallel to the raceway when the rotor is in the pump position and obliquely angled relative to the raceway when the rotor is in the tube load position.

In examples discussed further below, the arc of the raceway defines opposed arc ends, and the predetermined location of the roller corresponding to the angular position at which the motor is prevented from stopping is at an arc end. To this end, a controller can be configured for controlling the motor and an angular position sensor can be configured for sensing angular position of the motor and generating a signal indicative thereof to the controller. The controller is configured to use the signal to prevent the motor from stopping at the angular position in which at least one roller is in the predetermined location.

Also described herein is a method which includes rotating a peristaltic pump rotor relative to a raceway to urge fluid through a tube disposed between the raceway and the rotor. The method also includes automatically preventing the rotor from stopping at one or more predetermined angular positions.

In another aspect, a pump includes a raceway, a rotor spaced from the raceway, and a motor configured to rotate the rotor to urge fluid through a tube disposed between the raceway and the rotor. A controller is configured to control the motor to automatically prevent the motor from stopping at one or more predetermined angular positions.

In an example, a pump may have an arcuate raceway having a concave inner surface and a rotor facing the inner surface of the raceway. The rotor is rotatable relative to the raceway, and is translationally mounted relative to the raceway between a pump or operating position, wherein the rotor is spaced from the midpoint a first distance, and a tube load position, wherein the rotor is spaced from the midpoint a second distance greater than the first distance. A rotor motor is coupled to the rotor to rotate the rotor. One or more rollers are arranged on the rotor to contact tubing disposed between rotor and the raceway at least when the rotor is in the pump position. A loading motor may be coupled to the rotor to move the rotor translationally and/or rotationally between the pump position and the tube load position.

In examples, an operating element such as a button may be provided that is manipulable by a person or controller to energize the loading motor.

In another aspect, a pump assembly has a raceway, a rotor spaced from the raceway, and a rotor motor configured to rotate the rotor to urge fluid through a tube disposed between the raceway and the rotor. A loading motor is coupled to the rotor to move the rotor translationally and/or rotationally relative to the raceway.

The details of the present application, both as to its structure and operation, can best be understood in reference to the accompanying drawings, in which like reference numerals refer to like parts, and in which:

### BRIEF DESCRIPTION OF THE DRAWINGS

Figure 1 is a schematic view of a non-limiting system in accordance with the present invention;
Figure 2 is a perspective view of the pump with the rotor in the pump position;
Figure 3 is a top view of the pump with the rotor in the pump position;
Figure 4 is a perspective view of the pump with the rotor in the tube load position, illustrating a person manually loading the tube between the raceway and the rotor;
Figure 5 is a top view of the pump with the rotor in the tube load position;
Figures 6 and 7 are exploded perspective views from the bottom and top, respectively, of the pump, illustrating features of an example embodiment, with portions broken away in Figure 7;
Figures 8 and 9 are exploded side views respectively showing the relationship between the motor mount and the raceway in the tube load and pump positions of the rotor, with some portions broken away; and
Figure 10 is a perspective view of an alternate embodiment using a motor instead of a manually-operated handle with the rotor in the pump position, schematically illustrating the motor, gear, and operating button.

### DETAILED DESCRIPTION

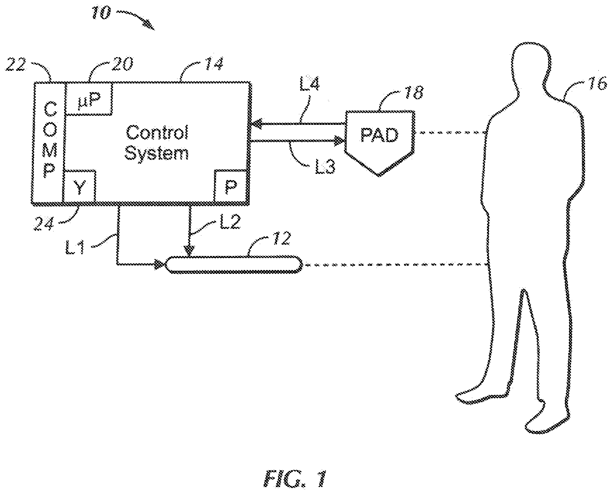

Referring initially to Figure 1, in accordance with present principles, a system 10 may include an intravascular heat exchange catheter 12 controlled by a control system 14 to control patient temperature, e.g., to prevent the patient 16 from becoming febrile or to induce therapeutic hypothermia in the patient 16. In the catheter, working fluid or coolant such as but not limited to saline circulates (typically under the influence of a pump "P" in the control system) in a closed loop from the control system 14, through a fluid supply line L1, through the catheter 12, and back to the system 14 through a fluid return line L2, such that no working fluid or coolant enters the body. While certain preferred catheters are disclosed herein, it is to be understood that other catheters can be used in accordance with present principles, including, without limitation, any of the catheters disclosed above or in the following U.S. patents: USPN 5,486,208, 5,837,003, 6,110,168, 6,149,673, 6,149,676, 6,231,594, 6,264,679, 6,306,161, 6,235,048, 6,238,428, 6,245,095, 6,251,129, 6,251,130, 6,254,626, 6,261,312, 6,312,452, 6,325,818, 6,409,747, 6,368,304, 6,338,727, 6,299,599, 6,287,326, 6,126,684, 7,211,106. The catheter 12 may be placed in the venous system, e.g., in the superior or inferior vena cava.

Instead of or in addition to the catheter 12, the system 10 may include one or more pads 18 that are positioned against the external skin of the patient 16 (only one pad 18 shown for clarity). The pad 18 may be, without limitation, any one of the pads disclosed in the external pad patents. The temperature of the pad 18 can be controlled by the control system 14 to exchange heat with the patient 16, including to induce therapeutic mild or moderate hypothermia in the patient in response to the patient presenting with, e.g., cardiac arrest, myocardial infarction, stroke, high intracranial pressure, traumatic brain injury, or other malady the effects of which can be ameliorated by hypothermia. The pad 18 may receive working fluid from the system 14 through a fluid supply line L3, and return working fluid to the system 14 through a fluid return line L4. The pump "P" may be a peristaltic pump which engages any one of the lines L1-L4, which are typically plastic IV lines, to urge working fluid through the lines through peristalsis.

The control system 14 may include one or more microprocessors 20 receiving target, and patient temperatures as input and controlling, among other things, the pump "P" and refrigerant compressor 22 and/or a bypass valve 24 that can be opened to permit refrigerant to bypass a condenser.

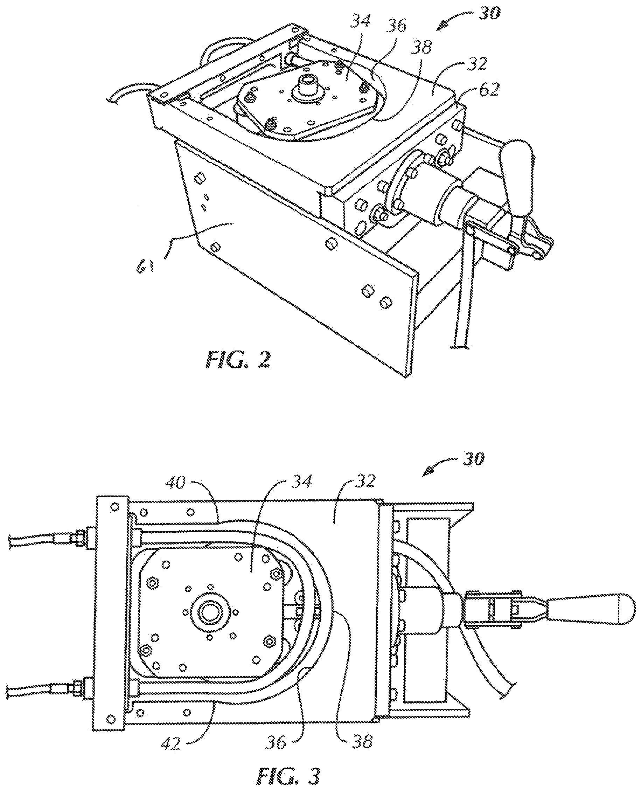

Turning now to Figures 2-5, an example of the pump "P" in Figure 1 is shown and generally designated 30. The pump 30 includes a rigid, preferably metal or hard plastic raceway 32 or channel and a rotor 34. The raceway 32 may be formed from one or more blocks of material as shown and has an inner arcuate surface 36 which may have a substantially constant radius of curvature. In some examples, the arcuate surface 36, which defines a midpoint 38 between its two ends 40, 42 (labeled in Figure 3), can extend through an arc of at least one hundred eighty degrees (180°) (e.g., where two drive rollers are used) and may extend through an arc of between one hundred eighty degrees (180°) and two hundred seventy degrees (270°), In the example shown, the arcuate surface 36 extends, from one end 40 to the other end 42, through an arc of greater than 200°. For example, the arc may be about 210° to 230°. In certain embodiments, the arcuate surface of a raceway may extend through an arc equal to 360° divided by n, where n is equal to the number of drive rollers mounted on or near a rotor which is rotated relative to the raceway,

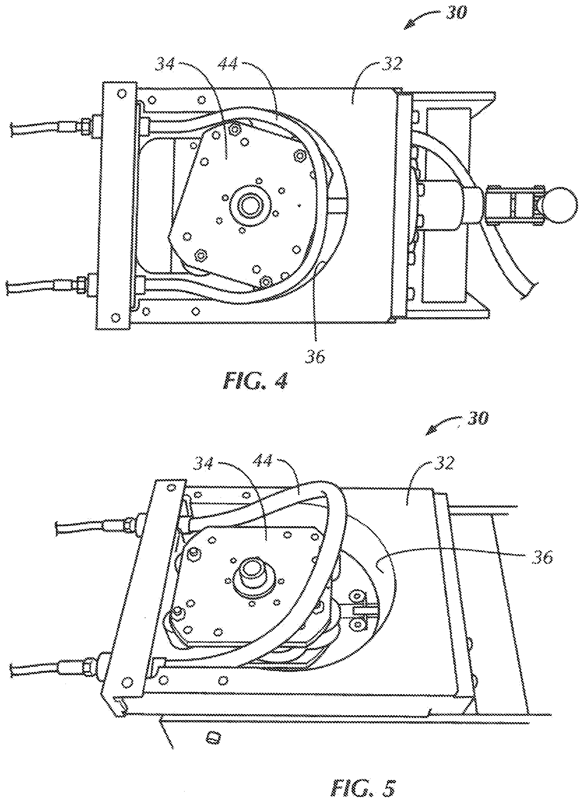

Covering more than 180° degrees of arc with the raceway may provide additional benefits, such as an extra margin against internal leakage. Furthermore, it is possible that covering more than 180° degrees of are allows the tubing to open gradually after being compressed by a drive roller and thereby reduce the degree of pulsatility of the flow. This in can reduce the amount of unwanted movement experienced by the downstream tubing and catheter when subject to pulsating flow. A motor, described further below, rotates the rotor 34 relative to the raceway 32. As well, the rotor 34 is movable translationally and/or rotationally relative to the raceway 32 between a pump position (Figures 2, 3, 6, 7, and 9), in which, the rotor 34 is spaced from the midpoint 38 of the inner surface 36 of the raceway 32 a first distance, and a tube load position (Figures 4, 5, and 8), in which the rotor 34 is spaced from the midpoint 38 a greater, second distance. As shown in Figures 2 and 3, in the pump position, rollers on the rotor 34 urge against a tube such as a pump tube or IV tube that is disposed between the rollers and the raceway 32. In the tube load position, the rotor 34 is sufficiently spaced from the raceway 32 to permit a tube 44 to be disposed between the raceway 32 and rotor 34 and to be removed therefrom, e.g., by hand. Example mechanisms for moving the rotor translationally and/or rotationally are discussed further below.

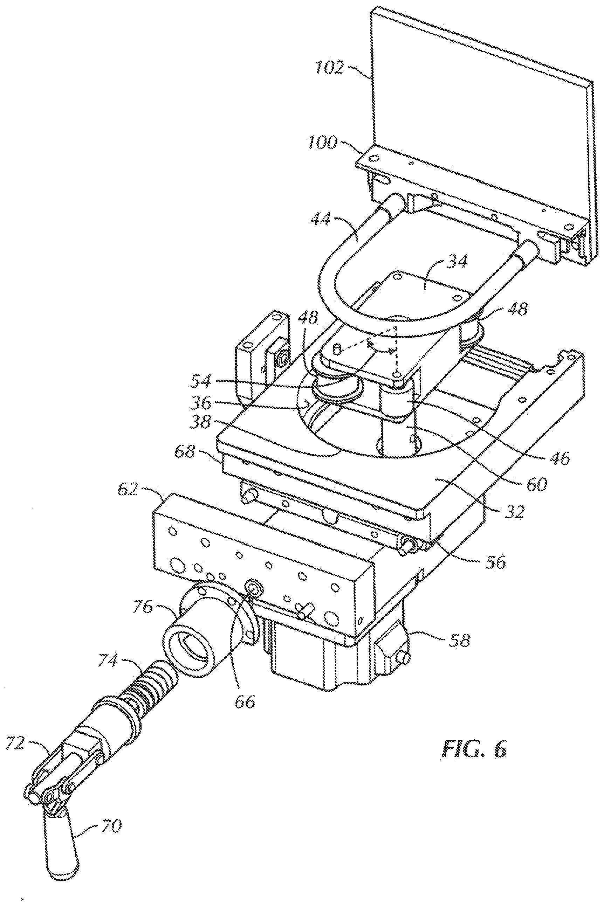

Indeed and now referring to Figures 6-9 for example structure, mounted on the rotor 34 are one or more rollers to urge against the tube 44 to pump fluid through the tube. In the example shown in Figure 6, the rotor 34 is defined in part by a rectilinear, non-square body, and on or near each corner of the body a roller is mounted, e.g., rotatably mounted to the rotor body. In the example, at one set of opposed corners on the body, drive rollers 46 are respectively mounted (only one drive roller shown in the perspective of Figure 6), whereas at the other set of opposed corners on the body, guide rollers 48 are respectively mounted. Thus, between the drive rollers 46 are guide rollers 48.

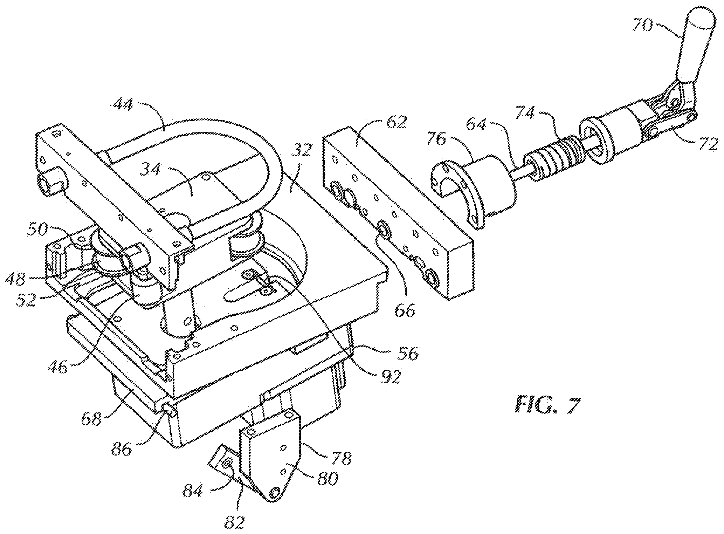

As shown in Figure 6, the drive roller 46 has a cylindrical or similar to cylindrical outer surface, and at least a portion of the outer surface is configured to urge against the tube 44. The outer surface of the example drive roller may be a single smooth cylinder and/or it may or may not have one or more flanges having peripheries extending beyond the cylindrical outer surface. In contrast, Figure 7 best shows that the guide roller 48 also has a cylindrical (or similar) outer surface but in addition includes top and/or bottom flanges 50, 52 defining respective peripheries extending beyond the cylindrical outer surface of the guide roller such that the tube 44 can be received on the cylindrical outer surface of the guide roller between the flanges 50, 52 when the rotor is in the pump position and is rotated. In the example shown, two and only two drive rollers 46 and two and only two guide rollers 48 are provided, but any number of drive and/or guide rollers may be utilized. In certain embodiments, the drive roller or guide roller may have a non-cylindrical or partially cylindrical outer surface.

Also, in the example shown, owing to the non-square shape of the rotor 34 body, the angle 54 between the drive roller 46 and guide roller 48 at one of the ends of the rotor body, with a vertex on a point on the roller body (e.g., the midpoint), is not ninety degrees. Instead, in the example shown, the angle 54 may be, for example, fifty five degrees. The same angle obtains at the opposite end of the rotor body. However, in some embodiments the rotor body is square, in which case all rollers are separated from the adjacent rollers by ninety degrees.

A block-like motor mount 56 supports a motor 58 such as a small ac or dc motor, in some embodiments, a stepper motor or other appropriate motor type. The motor 58 is coupled to the rotor 34 by an output shaft 60, with, in some embodiments, a reduction gear train (not shown) being meshed between the motor shaft and the output shaft 60.

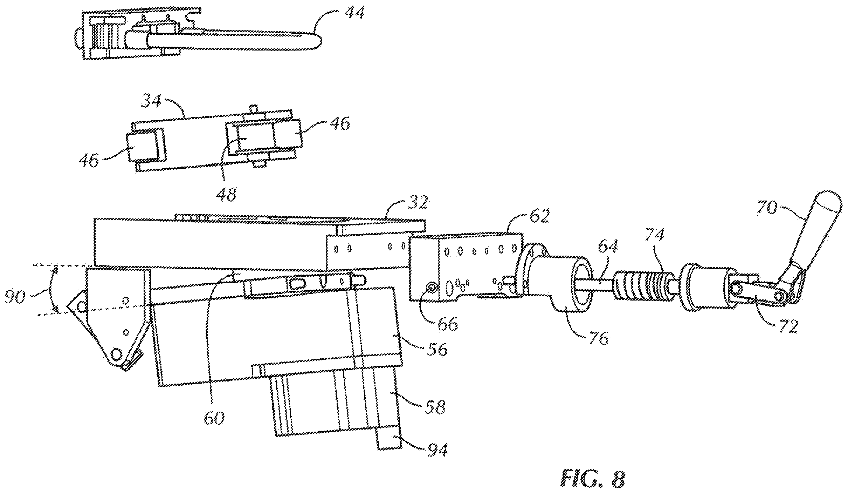

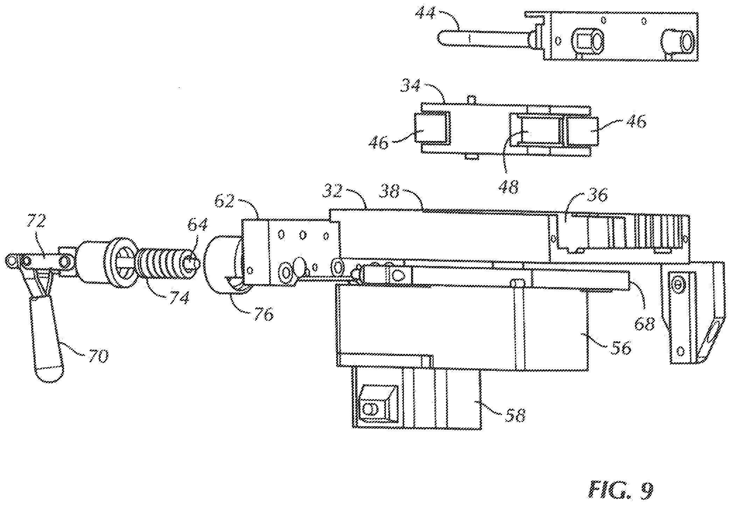

A positioning mechanism is coupled to the motor mount 56 and is manipulable by a person to move the motor mount 56 to thereby move the rotor 34 between the pump position and the tube load position. In a non-limiting example, referring briefly back to Figure 2, a base 61 stationarly bolds the raceway 32, and a rectilinear rigid support, block 62 (Figures 2 and 6-9) may be bolted or welded to the base 61 or made integrally therewith. A push rod 64 (Figures 7-9) extends through a hole 66 in the support block 62 to contact and/or be engaged with the motor mount 56 and/or with a motor plate 68 coupled to the motor mount 56. A handle 70 is coupled at a hinge mechanism 72 to the push rod 64. The handle 70 can be moved by hand to a substantially perpendicular orientation relative to the push rod 64 (Figures 6 and 9) to pull the push rod 64 and thus to move the motor mount 56 (and hence rotor 34) toward the inner surface of the raceway 32, thereby moving the rotor 34 to the pump position. The handle 70 can also be moved by hand down from the perpendicular orientation to the non-perpendicular orientation shown in Figures 7 and 8. This pushes the push rod 64 and thus moves the motor mount 56/rotor 34 away from the pump position to the tube load position. One or more radial bearings 74, 76 may be provided as appropriate to radially support elements of the positioning mechanism.

Also and focusing on Figure 7, to support the motor mount 56 and attendant elements that move with it, two side brackets 78 may be provided on respective sides of the raceway 32 (only one bracket 78 shown in Figure 7). A vertical flange 80 of the side bracket 78 may be affixed to the raceway 32, e.g., by threaded fasteners or welding, and a swing arm 82 pivotably coupled to the vertical flange 80 and rotatably coupled to the motor mount 56 or other component that moves with the motor mount 56. In the example shown in Figure 7, a hole 84 is formed in the swing arm 82 and rotatably engages a pin 86 that is attached to and that extends radially away from the motor plate 68. Recall that the motor mount 56, motor plate 68, and rotor 34 move translationally together as unit

Owing to the example positioning mechanism described above, as best shown in Figure 9 the motor mount 56 (with motor plate 68) is parallel to the raceway 32 when the rotor 34 is in the pump position. In contrast, as best shown in Figure 8, the motor mount 56 (with motor plate 68) is obliquely angled relative to the raceway 32 when the rotor 34 is in the tube load position. That is obliquely angled relative to the raceway 32 when the rotor 34 is in the tube load position. That is, an oblique angle 90 is established between, for example, the plane of the motor plate 68 and the plane defined by the bottom surface of the raceway 32 when the rotor 34 is in the tube load position, To further facilitate motion of the positioning mechanism when the handle 70 is moved, a hinge pin 92 (Figure 7) may be provided as part of the coupling between the push rod 64 and motor mount 56/motor plate 68.

Thus, the rotor 34 can move linearly relative to raceway 32. In the example shewn, linear bearings are used, it being understood that equivalently a multi-bar linkage between the rotor 34 and raceway 32 can be used for pseudo-linear motion. In any case, in the tube position the rotor 34 is a sufficient distance (typically an inch or more) so that the tube 44 can be inserted freely between the rotor 34 and raceway 32 by a person. Then, when the rotor is moved to the pump position, at least the drive rollers 46 urge into the tube 44 sufficiently to stretch the tube 44 by an elongation of at least 3% and typically 3-15%. This elongation advantageously ensures that slack does not build up in the tubing as it wears and stretches during use. As understood herein, such slack can lead to kinking of the tubing or excessive wear.

Figure 8 is used to schematically show that at least one angular position sensor 94 can be provided on the motor 58. Without limitation, the angular position sensor may be a Hall effect sensor, or a de stepper motor revolution counter, or a potentiometer type sensor. The sensor 94 generates an output representative of the angular position of the motor. The sensor 94 may be coupled to the motor shaft or the output shaft 60 or other part of the rotating mechanism in the pump. According to an embodiment of the invention,

the processor 20 shown in Figure 1 can control the motor 58 and can receive the signal from the sensor 94. Using the signal form the sensor 94, the processor 20 can prevent the motor 58 from stopping at an angular position corresponding to at least one roller 46/48 being in a predetermined angular location relative to the receway 32. In au example, the predetermined location of the roller corresponding to the angular position at which the motor is prevented form stopping is at an arc end 40 or 42 of the raceway 32. This ensures that, particularly when a raceway arc of >180 degrees is used, the rollers will not be in the 12 o'clock and 6 o'clock positions (i.e., adjacent to the ends of the arc), which would interfere with the raceway even when the rotor is in the tube load position and thereby complicate tube loading and unloading.

Thus, the position sensor 94 can be coupled to the motor shaft to indicate critical angular positions to avoid stopping the motor at these positions. According to an embodiment of the invention, the processor 20 can control the motor so that it will not stop on these critical positions. Alternately, the signal from the one or more sensors 94 can be used to indicate non-critical positions, with the processor 20 controlling the motor so it will always stop on these non-critical angular positions. Yet again, a mechanical means, mechanism or other element, e.g., a pin, may be used to ensure that the motor/rotor does not stop in critical positions.

Completing the description, the tube 44 may be configured as a loop as best shown in Figure 6, with the ends of the loop engaged with a manifold 100 in fluid communication with the interior of the manifold 10. In turn, the interior of the manifold 100 may communicate with a cassette 102 such as the cassette shown and described in U.S.patent publication US 2015/0230974 filed February 24, 2014. Such a cassette can be engaged with structure in the control system 14 to exchange heat with working fluid flowing through the cassette 102 and tube 44 and being circulated by the pump 30 shown and described herein to and from a heat exchange member such as the catheter 12 and/or pad 18.

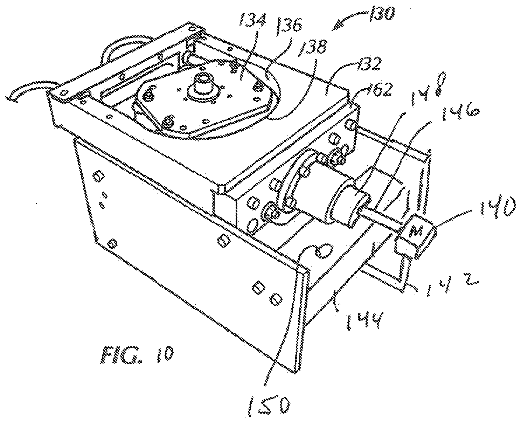

Figure 10 shows an alternate embodiment 130 of the peristaltic pump that in all essential respects is identical in configuration and operation to the pump 30 described above with the exceptions noted. Like the pump 30, the pump 130 in Figure 10 includes a rigid, preferably metal or hard plastic raceway 132 and a rotor 134. The raceway 132 or channel may be formed from one or more blocks of material as shown and has an inner arcuate surface 136 which may have a substantially constant radius of curvature. The arcuate surface 136 of Figure 10 is substantially identical in configuration and operation to the arcuate surface 32 in Figure 2. A motor rotates the rotor 134 relative to the raceway 132. As well, like the rotor 32 shown in Figure 2, the rotor 134 shown in Figure 10 may be movable translationally and/or rotationally relative to the raceway 132 between a pump position, in which the rotor 134 is spaced from the midpoint 138 of the inner surface 136 of the raceway 132 a first distance, and a tube load position in which the rotor 134 is spaced from the midpoint 138 a greater, second distance.

However, unlike the pump 30 shown in Figure 2, the pump 130 shown in Figure 10 provides a loading motor 140 to provide for automated or automatic movement of the rotor relative to the raceway, e.g., translational or rotational, instead of a manuallymanipulable handle. Thus, the pump 130 in Figure 10 omits, e.g., the handle 70 with hinge mechanism 72.

Instead, the loading motor 140, which may be mounted (e.g., directly on or by means of a bracket 142) to a pump base 144, reciprocatingly drives a push rod 146 to move the rotor 134 between the pump position and tube load position. Advantages associated with using a loading motor to move the rotor include but are not limited to the following: providing convenience for the customer, e.g., the customer may not have to reach back and apply force to move the motor; the customer may not have access to moving parts; minimal or no room for the customer's hand may be needed, which in turn saves space; movement of the rotor may be controlled or allowed only when certain other conditions are met; the rotor may be moved with constant speed; and the force needed to move the rotor may be monitored. The motor 140 may be a direct current, (dc) stepper motor or other ac or dc motor, or other appropriate motor type and the push rod 146 may be the rack element of a rack-and-pinion gear, with the pinion portion being geared to a shaft rotated by the motor 140. The push rod 146 may extend through a support block 162 that is substantially identical in configuration and operation to the support block 62 shown in Figure 6 to contact and/or be engaged with a motor mount that supports the motor that rotates the rotor 134. One or more radial bearings 148 may be provided as appropriate to radially support elements of the positioning mechanism.

An operating button or key 150 may be manipulable by a person or controller to energize the loading motor 140. The button or key 150 may be positioned on the pump 130 base as shown and may be electrically connected to the controller of the motor 140, with the motor and its controller enclosed in the rectangular box shown at 140. In all other essential respects the pump 130 shown in Figure 10 may be substantially identical in configuration and operation to the pump 30 shown in Figures 2-9.

While the particular EASY LOADING HIGH PERFORMANCE PERISTALTIC PUMP is herein shown and described in detail, the scope of the present invention is to be limited by nothing other than the appended claims.

## Claims

1. A pump assembly, comprising:
a raceway (32, 130);
a rotor (34, 134) spaced from the raceway (32, 130);
a rotor motor (58) configured to rotate the rotor (34, 134) to urge fluid through a tube (44) disposed between the raceway (32, 130) and the rotor (34, 134);
a loading motor (140) coupled to the rotor (34, 134) to move the rotor (134) translationally and/or rotationally relative to the raceway (130) between a pump position and a tube load position; and a controller configured to automatically prevent the rotor (34) from stopping at one or more predetermined angular positions in which the rotor (34, 134) would interfere with the raceway (32, 130) when the rotor is in the tube load position and thereby complicate tube loading and unloading, or to control the motor so it will always stop on non-critical angular positions in which the rotor (34, 134) would not interfere with the raceway (32, 130) when the rotor is in the tube load position and thereby complicate tube loading and unloading.

2. The pump assembly of Claim 1, wherein the raceway (32, 130) has an inner surface (36, 136) extending through an arc of at least one hundred eighty degrees (180°), the arc defining a midpoint (38, 138), and the rotor (34, 134) faces the inner surface (36, 136) of the raceway (32, 130).

3. The pump assembly of Claim 1 or claim 2, wherein the rotor (34, 134) is rotatable relative to the raceway (32, 130) and is also translationally mounted relative to the raceway (32, 130) between the pump position, wherein the rotor (34, 134) is spaced from the midpoint (38) a first distance, and the tube load position, wherein the rotor (34, 134) is spaced from the midpoint a second distance greater than the first distance.

4. The pump assembly of Claim 1, wherein an inner surface (136) of the raceway (130) extends through an arc of at least one hundred eighty degrees (180°).

5. The pump assembly of any preceding claim, comprising:
at least one drive roller (46) on the rotor (34, 134), the drive roller having a cylindrical outer surface, at least a portion of the outer surface being configured to urge against a tube disposed between the rotor (34, 134) and the raceway (32, 130), the drive roller having no flanges with peripheries extending beyond the cylindrical outer surface; and
at least one guide roller (48) on the rotor (34, 134), the guide roller having a cylindrical outer surface and top and bottom flanges (50, 52) defining respective peripheries extending beyond the cylindrical outer surface of the guide roller such that a tube disposed between the rotor (34, 134) and the raceway (32, 130) is receivable on the cylindrical outer surface of the guide roller between the flanges.

6. The pump assembly of any preceding claim, comprising an operating element (150) manipulable by a person or controller to energize the loading motor (140).

7. The pump assembly of claim 5 wherein:
the raceway is an arcuate raceway (32/130) having a concave inner surface (36/136) extending through an arc of at least one hundred eighty degrees (180°), the arc defining a midpoint (38/138);
the rotor (34/134) faces the inner surface (36/136) of the raceway (32/130), the rotor (34/134) being rotatable relative to the raceway (32/130), the rotor (34/134) being translationally mounted relative to the raceway (32/130) between the pump position, wherein the rotor (34/134) is spaced from the midpoint (38/138) a first distance, and the tube load position, wherein the rotor (34/134) is spaced from the midpoint (38/138) a second distance greater than the first distance; and
said at least one drive roller (46) and/or said at least one guide roller (48) comprises plural rollers (46, 48) arranged on the rotor (34/134) to contact tubing disposed between the rotor (34/134) and the raceway (32/130) at least when the rotor (34/134) is in the pump position, wherein the motor (58) is prevented from stopping at at least one angular position in which at least one roller (46/48) is in a predetermined location.

8. The pump assembly of Claim 7, wherein the concave inner surface (36/136) of the raceway (32/130) extends through an arc of between one hundred eighty degrees (180°) and two hundred degrees (200°).

9. The pump of Claim 5 wherein said at least one drive roller (46) and / or said at least one guide roller (48) comprises either
(a) plural drive rollers (46) and / or plural guide rollers, or
(b) two and only two drive rollers (46) and two and only two guide rollers (48).

10. The pump assembly of Claim 5, comprising a motor mount (56) supporting the motor and rotor (34) and a positioning mechanism (70) coupled to the motor mount (56) and manipulable by a person to move the motor mount (56) to thereby move the rotor (34) between the pump position and the tube load position.

11. The pump assembly of Claim 10, wherein the motor mount (56) is parallel to the raceway (32) when the rotor (34) is in the pump position, the motor mount (56) being obliquely angled relative to the raceway (32) when the rotor (34) is in the tube load position.

12. The pump assembly of Claim 5, wherein the loading motor (140) is energizable to move the rotor (134) between the pump position and the tube load position.

13. The pump assembly of Claim 5, wherein the arc defines opposed arc ends (40, 42), and the predetermined location of the roller (46/48) corresponding to the angular position at which the motor (58) is prevented from stopping is at an arc end (40/42).

14. The pump assembly of Claim 5, comprising a controller (20) configured for controlling the motor (58) and an angular position sensor (94) configured for sensing angular position of the motor (58) and generating a signal indicative thereof to the controller (20), the controller (20) configured to use the signal to prevent the motor (58) from stopping at the angular position in which at least one roller (46/48) is in the predetermined location.

## Patentansprüche

1. Pumpenanordnung, die Folgendes umfasst:
eine Laufbahn (32, 130);
einen Rotor (34, 134), der von der Laufbahn (32, 130) beabstandet ist;
einen Rotormotor (58), der konfiguriert ist, um den Rotor (34, 134) zu rotieren, um Fluid durch ein Rohr (44) zu drängen, das zwischen der Laufbahn (32, 130) und dem Rotor (34, 134) angeordnet ist;
einen Lademotor (140), der mit dem Rotor (34, 134) gekoppelt ist, um den Rotor (134) relativ zu der Laufbahn (130) zwischen einer Pumpenposition und einer Rohrladeposition translatorisch und/oder rotativ zu bewegen; und eine Steuervorrichtung, die konfiguriert ist, um den Rotor (34) automatisch davon abzuhalten, an einer oder mehreren zuvor bestimmten Winkelpositionen anzuhalten, in denen der Rotor (34, 134) die Laufbahn (32, 130) stören würde, wenn sich der Rotor in der Rohrladeposition befindet, und wobei dadurch das Laden und Entladen des Rohrs kompliziert würde oder um den Motor zu steuern, dass er immer an nicht kritischen Winkelpositionen anhält, in denen der Rotor (34, 134) die Laufbahn (32, 130) nicht stören würde, wenn sich der Rotor in der Rohrladeposition befindet und wobei dadurch das Laden und Entladen des Rohrs nicht kompliziert würde.

2. Pumpenanordnung nach Anspruch 1, wobei die Laufbahn (32, 130) eine Innenoberfläche (36, 136) aufweist, die sich durch einen Bogen von wenigstens einhundertachtzig Grad (180°) erstreckt, wobei der Bogen einen Mittelpunkt (38, 138) definiert und der Rotor (34, 134) der Innenoberfläche (36, 136) der Laufbahn (32, 130) zugewandt ist.

3. Pumpenanordnung nach Anspruch 1 oder 2, wobei der Rotor (34, 134) relativ zu der Laufbahn (32, 130) rotierbar ist und ebenso relativ zu der Laufbahn (32, 130) zwischen der Pumpenposition, wobei der Rotor (34, 134) von dem Mittelpunkt (38) mit einer ersten Entfernung beabstandet ist, und der Rohrladeposition, wobei der Rotor (34, 134) von dem Mittelpunkt mit einer zweiten Entfernung beabstandet ist, die größer als die erste Entfernung ist, translatorisch gelagert ist.

4. Pumpenanordnung nach Anspruch 1, wobei sich eine Innenoberfläche (136) der Laufbahn (130) durch einen Bogen von wenigstens einhundertachtzig Grad (180°) erstreckt.

5. Pumpenanordnung nach einem der vorhergehenden Ansprüche, die Folgendes umfasst:
wenigstens eine Antriebsrolle (46) an dem Rotor (34, 134), wobei die Antriebsrolle eine zylindrische Außenoberfläche aufweist, wobei wenigstens ein Abschnitt der Außenoberfläche konfiguriert ist, um gegen ein Rohr zu drängen, das zwischen dem Rotor (34, 134) und der Laufbahn (32, 130) angeordnet ist, wobei die Antriebsrolle keine Flansche mit Umfängen aufweist, die sich über die zylindrische Außenoberfläche hinaus erstrecken; und
wenigstens eine Führungsrolle (48) an dem Rotor (34, 134), wobei die Führungsrolle eine zylindrische Außenoberfläche und obere und untere Flansche (50, 52) aufweist, die jeweilige Umfänge definieren, die sich über die zylindrische Außenoberfläche der Führungsrolle derart hinaus erstrecken, dass ein Rohr, das zwischen dem Rotor (34, 134) und der Laufbahn (32, 130) angeordnet ist, auf der zylindrischen Außenoberfläche der Führungsrolle zwischen den Flanschen aufgenommen werden kann.

6. Pumpenanordnung nach einem der vorhergehenden Ansprüche, die ein Bedienelement (150) umfasst, das mittels einer Person oder einer Steuervorrichtung betätigt werden kann, um den Lademotor (140) mit Strom zu versorgen.

7. Pumpenanordnung nach Anspruch 5, wobei:
die Laufbahn eine bogenförmige Laufbahn (32/130) ist, die eine konkave Innenoberfläche (36/136) aufweist, die sich durch einen Bogen von wenigstens einhundertachtzig Grad (180°) erstreckt, wobei der Bogen einen Mittelpunkt (38/138) definiert;
der Rotor (34/134) der Innenoberfläche (36/136) der Laufbahn (32/130) zugewandt ist, wobei der Rotor (34/134) relativ zu der Laufbahn (32/130) rotierbar ist, wobei der Rotor (34/134) relativ zu der Laufbahn (32/130) zwischen der Pumpenposition, wobei der Rotor (34/134) von dem Mittelpunkt (38/138) mit einer ersten Entfernung beabstandet ist, und der Rohrladeposition, wobei der Rotor (34/134) von dem Mittelpunkt (38/138) mit einer zweiten Entfernung, die größer als die erste Entfernung ist, translatorisch gelagert ist; und
die wenigstens eine Antriebsrolle (46) und/oder die wenigstens eine Führungsrolle (48) mehrere Rollen (46, 48) umfasst, die an dem Rotor (34/134) eingerichtet sind, um Rohre zu berühren, die zwischen dem Rotor (34/134) und der Laufbahn (32/130) angeordnet sind, wenigstens dann, wenn sich der Rotor (34/134) in der Pumpenposition befindet, wobei der Motor (58) davon abgehalten wird, an wenigstens einer Winkelposition anzuhalten, in der sich wenigstens eine Rolle (46/48) an einer zuvor bestimmten Stelle befindet.

8. Pumpenanordnung nach Anspruch 7, wobei sich die konkave Innenoberfläche (36/136) der Laufbahn (32/130) durch einen Bogen von zwischen einhundertachtzig Grad (180°) und zweihundert Grad (200°) erstreckt.

9. Pumpe nach Anspruch 5, wobei die wenigstens eine Antriebsrolle (46) und/oder die wenigstens eine Führungsrolle (48) Folgendes umfassen, entweder
(a) mehrere Antriebsrollen (46) und/oder mehrere Führungsrollen, oder
(b) zwei und nur zwei Antriebsrollen (46) und zwei und nur zwei Führungsrollen (48).

10. Pumpenanordnung nach Anspruch 5, die eine Motorlagerung (56), die den Motor und den Rotor (34) trägt, und einen Positionierungsmechanismus (70) umfasst, der mit der Motorlagerung (56) gekoppelt ist und mittels einer Person betätigt werden kann, um die Motorlagerung (56) zu bewegen, um dadurch den Rotor (34) zwischen der Pumpenposition und der Rohrladeposition zu bewegen.

11. Pumpenanordnung nach Anspruch 10, wobei die Motorlagerung (56) parallel zu der Laufbahn (32) ist, wenn sich der Rotor (34) in der Pumpenposition befindet, wobei die Motorlagerung (56) relativ zu der Laufbahn (32) schräg abgewinkelt ist, wenn sich der Rotor (34) in der Rohrladeposition befindet.

12. Pumpenanordnung nach Anspruch 5, wobei der Lademotor (140) mit Strom versorgt werden kann, um den Rotor (134) zwischen der Pumpenposition und der Rohrladeposition zu bewegen.

13. Pumpenanordnung nach Anspruch 5, wobei der Bogen gegenüberliegende Bogenenden (40, 42) definiert und die zuvor bestimmte Stelle der Rolle (46/48), die der Winkelposition entspricht, an der der Motor (58) davon abgehalten wird, anzuhalten, an einem Bogenende (40/42) ist.

14. Pumpenanordnung nach Anspruch 5, die eine Steuervorrichtung (20), die zum Steuern des Motors (58) konfiguriert ist, und einen Winkelpositionssensor (94) umfasst, der zum Erfassen der Winkelposition des Motors (58) und zum Erzeugen eines dafür bezeichnenden Signals an die Steuervorrichtung (20) konfiguriert ist, wobei die Steuervorrichtung (20) konfiguriert ist, um das Signal zu verwenden, um den Motor (58) davon abzuhalten an der Winkelposition anzuhalten, in der sich wenigstens eine Rolle (46/48) an der zuvor bestimmten Stelle befindet.

## Revendications

1. Ensemble pompe, comprenant :
un chemin de roulement (32, 130) ;
un rotor (34, 134) espacé du chemin de roulement (32, 130) ;
un moteur de rotor (58) conçu pour mettre le rotor (34, 134) en rotation afin de pousser du fluide à travers un tube (44) disposé entre le chemin de roulement (32, 130) et le rotor (34, 134) ;
un moteur de chargement (140) accouplé au rotor (34, 134) pour déplacer le rotor (134) en translation et/ou en rotation par rapport au chemin de roulement (130) entre une position de pompe et une position de charge de tube ; et un dispositif de commande configuré pour que le rotor (34) soit automatiquement dans l'impossibilité de s'arrêter au niveau d'une ou plusieurs positions angulaires prédéterminées dans lesquelles le rotor (34, 134) interfère avec le chemin de roulement (32, 130) lorsque le rotor se trouve dans la position de charge de tube et complique ainsi le chargement et le déchargement de tube, ou pour commander le moteur de sorte qu'il s'arrête toujours sur des positions angulaires non critiques dans lesquelles le rotor (34, 134) n'interfère pas avec le chemin de roulement (32, 130) lorsque le rotor se trouve dans la position de charge de tube et ne complique donc pas le chargement et le déchargement du tube.

2. Ensemble pompe selon la revendication 1, le chemin de roulement (32, 130) ayant une surface interne (36, 136) s'étendant à travers un arc d'au moins cent quatre-vingts degrés (180°), l'arc définissant un point médian (38, 138), et le rotor (34, 134) faisant face à la surface interne (36, 136) du chemin de roulement (32, 130).

3. Ensemble pompe selon la revendication 1 ou la revendication 2, le rotor (34, 134) pouvant être en rotation par rapport au chemin de roulement (32, 130) et étant également monté en translation par rapport au chemin de roulement (32, 130) entre la position de la pompe, le rotor (34, 134) étant espacé du point médian (38) d'une première distance, et la position de charge de tube, le rotor (34, 134) étant espacé du point médian d'une seconde distance supérieure à la première distance.

4. Ensemble pompe selon la revendication 1, une surface interne (136) du chemin de roulement (130) s'étendant à travers un arc d'au moins cent quatre-vingts degrés (180°).

5. Ensemble pompe selon l'une quelconque des revendications précédentes, comprenant :
au moins un rouleau d'entraînement (46) sur le rotor (34, 134), le rouleau d'entraînement ayant une surface externe cylindrique, au moins une partie de la surface externe étant conçue pour pousser contre un tube disposé entre le rotor (34, 134) et le chemin de roulement (32, 130), le rouleau d'entraînement ne comprenant pas de brides avec des périphéries s'étendant au-delà de la surface externe cylindrique ; et
au moins un rouleau de guidage (48) sur le rotor (34, 134), le rouleau de guidage ayant une surface externe cylindrique et des brides supérieure et inférieure (50, 52) définissant des périphéries respectives s'étendant au-delà de la surface externe cylindrique du rouleau de guidage de telle sorte qu'un tube disposé entre le rotor (34, 134) et le chemin de roulement (32, 130) peut être reçu sur la surface externe cylindrique du rouleau de guidage entre les brides.

6. Ensemble pompe selon l'une quelconque des revendications précédentes, comprenant un élément de fonctionnement (150) pouvant être manipulé par une personne ou un dispositif de commande pour activer le moteur de chargement (140).

7. Ensemble pompe selon la revendication 5 :
le chemin de roulement étant un chemin de roulement arqué (32/130) ayant une surface interne concave (36/136) s'étendant à travers un arc d'au moins cent quatre-vingts degrés (180°), l'arc définissant un point médian (38/138) ;
le rotor (34/134) faisant face à la surface interne (36/136) du chemin de roulement (32/130), le rotor (34/134) pouvant être en rotation par rapport au chemin de roulement (32/130), le rotor (34/134) étant monté en translation par rapport au chemin de roulement (32/130) entre la position de pompe, le rotor (34/134) étant espacé du point médian (38/138) d'une première distance, et la position de charge de tube, le rotor (34/134) étant espacé du point médian (38/138) d'une seconde distance supérieure à la première distance ; et
ledit au moins un rouleau d'entraînement (46) et/ou ledit au moins un rouleau de guidage (48) comprenant plusieurs rouleaux (46, 48) disposés sur le rotor (34/134) pour entrer en contact avec une tubulure disposée entre le rotor (34/134) et le chemin de roulement (32/130) au moins lorsque le rotor (34/134) se trouve dans la position de pompe, le moteur (58) étant dans l'impossibilité de s'arrêter au niveau d'au moins une position angulaire dans laquelle au moins un rouleau (46/48) se trouve à un emplacement prédéterminé.

8. Ensemble pompe selon la revendication 7, la surface interne concave (36/136) du chemin de roulement (32/130) s'étendant à travers un arc compris entre cent quatre-vingts degrés (180°) et deux-cent degrés (200°).

9. Pompe selon la revendication 5, ledit au moins un rouleau d'entraînement (46) et/ou ledit au moins un rouleau de guidage (48) comprenant soit
(a) plusieurs rouleaux d'entraînement (46) et/ou plusieurs rouleaux de guidage, soit
(b) deux et seulement deux rouleaux d'entraînement (46) et deux et seulement deux rouleaux de guidage (48).

10. Ensemble pompe selon la revendication 5, comprenant un support de moteur (56) supportant le moteur et le rotor (34) et un mécanisme de positionnement (70) accouplé au support de moteur (56) et pouvant être manipulé par une personne pour déplacer le support de moteur (56) pour déplacer ainsi le rotor (34) entre la position de pompe et la position de charge de tube.

11. Ensemble pompe selon la revendication 10, le support de moteur (56) étant parallèle au chemin de roulement (32) lorsque le rotor (34) se trouve dans la position de pompe, le support de moteur (56) étant incliné obliquement par rapport au chemin de roulement (32) lorsque le rotor (34) se trouve en position de charge de tube.

12. Ensemble pompe selon la revendication 5, le moteur de chargement (140) pouvant être activé pour déplacer le rotor (134) entre la position de pompe et la position de charge de tube.

13. Ensemble pompe selon la revendication 5, l'arc définissant des extrémités d'arc opposées (40, 42), et l'emplacement prédéterminé du rouleau (46/48) correspondant à la position angulaire au niveau de laquelle le moteur (58) est dans l'impossibilité de s'arrêter se trouvant au niveau d'une extrémité d'arc (40/42).

14. Ensemble pompe selon la revendication 5, comprenant un dispositif de commande (20) configuré pour commander le moteur (58) et un capteur de position angulaire (94) configuré pour détecter la position angulaire du moteur (58) et générer un signal indiquant celle-ci au dispositif de commande (20), le dispositif de commande (20) étant configuré pour utiliser le signal afin que le moteur (58) soit dans l'impossibilité de s'arrêter au niveau de la position angulaire dans laquelle au moins un rouleau (46/48) se trouve à l'emplacement prédéterminé.
